# EUROPEAN PATENT APPLICATION

(11) **EP 1 550 670 A1**
(43) Date of publication of application: **06.07.2005**
(21) Application number: 03753983.0
(22) Date of filing: 02.10.2003
(51) Int. Cl.: C07K 5/00, C07K 1/04, A61K 47/48, A61K 9/50, A61K 7/00

(54) **FINE SPHERICAL PARTICLES WITH SATISFACTORY MOLECULAR ORIENTATION, SPHERICAL MICROCAPSULES COMPRISING THE SAME, AND PROCESSES FOR PRODUCING THESE**

(30) Priority: 07.10.2002 JP 2002293533; 05.06.2003 JP 2003160291
(71) Applicant: National Institute of Advanced Industrial Science and Technology, Tokyo 100-8921 (JP)
(72) Inventor: MATSUZAWA, Yoko, Tsukuba-shi, Ibaraki (JP); MATSUMOTO, Mutsuyoshi, Tsukuba-shi, Ibaraki (JP); KOGISO, Masaki, Tsukuba-shi, Ibaraki (JP); SHIMIZU, Toshimi, Tsukuba-shi, Ibaraki (JP)
(74) Representative: Zimmermann, Gerd Heinrich
(86) International application number: PCT/JP2003/012636
(87) International publication number: WO 2004/031214

(57) **Abstract**

The present invention relates to a fine spherical particle having uniform molecular orientation having uniform molecular orientation, which is useful in fine chemical fields or electronic and information fields, such as a functional material and a medical material, using membrane formation of a bicephalic compound; a spherical microcapsule encapsulating a hydrophilic core substance; and a process for producing the same. The fine spherical particle can be produced by immersing a substrate having hydrophilicity in an aqueous solution of a salt of the compound represented by the following formula (1) to precipitate the fine particle under an acidic atmosphere. 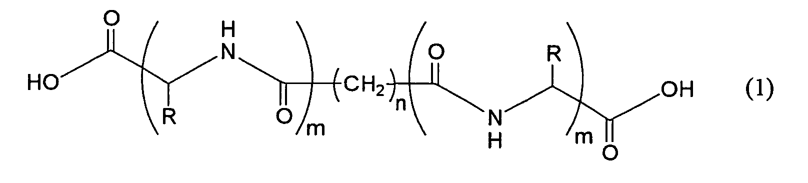 wherein R represents a hydrogen atom or an alkyl group having 1 to 5 carbon atoms; n is an integer of 8 to 20; and m is an integer of 1 to 3. The spherical microcapsule encapsulating a fine particle of a hydrophilic core substance is produced by immersing a substrate having hydrophilicity in an aqueous solution in which a metal salt of the compound represented by formula (1) and the hydrophilic core substance are dissolved; and allowing the aqueous solution to stand under an acidic atmosphere to precipitate the fine particle. The resulting fine spherical particle and the spherical microcapsule has a particle diameter of 0.01 to 100 µm.

## Description

### Technical Field

The present invention relates to a fine spherical particle having uniform molecular orientation, comprising a low-molecular-weight organic compound, which is useful in fine chemical fields and electronic and information fields, such as a functional material and a medical material; a microcapsule stably encapsulating a hydrophilic core substance inside the fine particle; and a process for producing the same.

### Background Art

The compound represented by the following formula (1) shows a remarkable properties as a bicephalic lipid having amphiphilicity and is expected to be utilized for various applications in fine chemical fields and electronic and information fields, such as a functional material and a medical material.

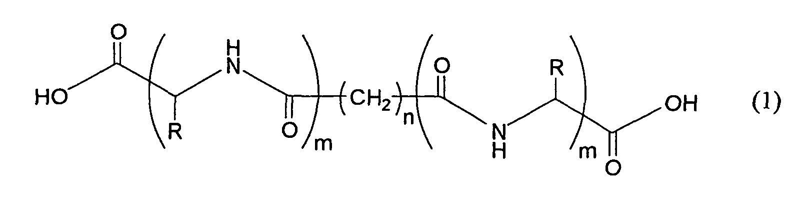

In formula (1), R represents a hydrogen atom or an alkyl group having 1 to 5 carbon atoms; n is an integer of 8 to 20; and m is an integer of 1 to 3.

It is described, for example, in Japanese Patent No. 3012932 and *Chem. Comm.,* 1998, pp. 1791-1792 that the above compound forms a nano-scale fiber having a width of about 10 to 30 nm when an aqueous alkaline solution of the compound is gradually acidified.

On the other hand, recently, microcapsules have been actively researched and developed on their applicability in wide fields of electronic and information materials such as recording materials and displaying materials, pharmaceuticals, functional materials, industrial materials, agricultural materials, fragrant materials, cosmetics and foods, and have already been in practical use or in practical stage in several fields.

In this connection, as representative technologies for producing microcapsules, there are known (1) chemical methods such as an interfacial polymerization method and an *in-situ* polymerization method, (2) physicochemical methods such as a coacervation method, an interfacial precipitation method, a submerged drying method and an orifice method, and (3) mechanical methods such as a dry mixing method and a spray drying method.

In the above methods of (1), membranes are formed by mainly using polymerization reactions and, in the methods of (2), membranes are formed by using phase-separation methods. However, it is difficult to encapsulate a hydrophilic core substance stably in the microcapsules obtained by these methods.

Recently, as a method for producing a microcapsule encapsulating a hydrophilic core substance, a method using a gel emulsion method has been proposed, for example, in JP-A-10-83340, but this method has problems that it requires many production steps and is complicated.

Moreover, as a method for producing fine fibers using a bicephalic compound, for example, a method described in JP-A-11-322787 has been proposed; and as a method for obtaining a fine spherical particle of a bicephalic compound, for example, a method described in Japanese Patent Application No. 2002-293533 has been proposed. However, membrane formation using these bicephalic compounds has not at all been known.

Furthermore, in the case of applying these bicephalic compounds to fine chemical fields and electronic and information fields such as functional materials and medical materials, it is necessary to convert the compounds into fine particles having uniform properties but fine particles having uniform properties comprising the above compounds have not been known.

Accordingly, an object of the present invention is to provide a fine spherical particle having uniform molecular orientation, comprising the compound represented by the above formula (1), which is useful in fine chemical fields and electronic and information fields; and a process for producing the same.

Furthermore, another object of the present invention is to provide a process for producing a spherical microcapsule encapsulating a hydrophilic core substance utilizing membrane formation with the compound represented by formula (1).

### Disclosure of the Invention

As a result of extensive studies on chemical properties of bicephalic peptide lipids, the present inventors have found that a specific bicephalic amphiphilic compound self-assembles under specific conditions and forms a spherical membrane to give a fine spherical particle having uniform molecular orientation and, when a metal salt of the compound and a hydrophilic core substance are used, the compound self-assembled under specific conditions to give a spherical microcapsule encapsulating a fine particle of the hydrophilic core substance. Thus, they have accomplished the present invention.

Specifically, the present invention relates to the followings:
1. A fine spherical particle having uniform molecular orientation, which comprises a compound represented by the following formula (1): 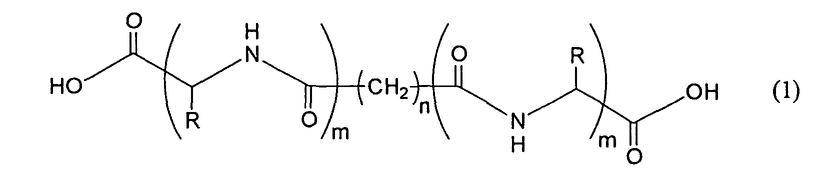 wherein R represents a hydrogen atom or an alkyl group having 1 to 5 carbon atoms; n is an integer of 8 to 20; and m is an integer of 1 to 3.
2. The fine spherical particle according to the above 1, wherein the fine particle is evenly oriented in a radial pattern from the center.
3. The fine spherical particle according to the above 1 or 2, wherein the particle diameter of the fine particle is from 0.01 to 100 µm.
4. A process for producing the fine spherical particle according to any one of the above 1 to 3, which comprises immersing a substrate having hydrophilicity in an aqueous solution of a salt of the compound represented by formula (1) to precipitate the fine particle under an acidic atmosphere.
5. The process for producing the fine spherical particle according to the above 4, wherein the salt of the compound represented by formula (1) is an alkali metal salt.
6. The process for producing the fine spherical particle according to the above 4 or 5, wherein the substrate comprises glass, metal, silica, mica, ceramic, earthenware, porcelain, plastic, or a composite material thereof.
7. The process for producing the fine spherical particle according to any one of the above 4 to 6, wherein the fine particle is precipitated under an acidic atmosphere of pH 5 to 6.
8. A spherical microcapsule in which a fine particle of a hydrophilic core substance is encapsulated inside the spherical body of the compound represented by formula (1) having uniform molecular orientation.
9. The spherical microcapsule according to the above 8, wherein the spherical microcapsule has a particle diameter of from 0.01 to 100 µm.
10. A process for producing the spherical microcapsule encapsulating a fine particle of a hydrophilic core substance according to the above 8 or 9, which comprises immersing a hydrophilicity-treated substrate in an aqueous solution in which a metal salt of the compound represented by formula (1) and the hydrophilic core substance are dissolved; and allowing the aqueous solution to stand under an acidic atmosphere for precipitation.
11. The process for producing the spherical microcapsule according to the above 10, wherein the metal salt of the compound represented by formula (1) is an alkali metal salt.
12. The process for producing the spherical microcapsule according to the above 10 or 11, wherein the acidic atmosphere is a weakly acidic atmosphere of pH 5 to 6.
13. The process for producing the spherical microcapsule according to any one of the above 10 to 12, wherein the substrate is selected from glass, metal, silica, mica, a ceramic, earthenware, porcelain, plastic, and a composite material thereof.

### Brief Description of the Drawings

Fig. 1 is a schematic drawing showing a step of producing the fine spherical particle having uniform molecular orientation or the spherical microcapsule of the present invention. Fig. 2 is a schematic drawing showing states of molecular orientation of the fine spherical particle of the present invention.
Fig. 3 is a conceptual drawing showing a production process of the microcapsule of the present invention, and Fig. 4 is a fluorescent microscopic photograph of the microcapsule obtained by the present invention.

### Symbols:

- 1:: hydrophilicity-treated substrate
- 2:: aqueous solution containing compound (1) or aqueous solution containing compound (1) and hydrophilic core substance
- 3:: vessel containing aqueous solution
- 4:: aqueous acidic solution
- 5:: vessel containing aqueous acidic solution
- 6:: lid

### Best Mode for Carrying Out the Invention

The present invention provides a fine spherical particle having uniform molecular orientation or a spherical microcapsule utilizing membrane formation by self-assembly of a bicephalic amphiphilic compound represented by the above formula (1).

### Fine spherical particle:

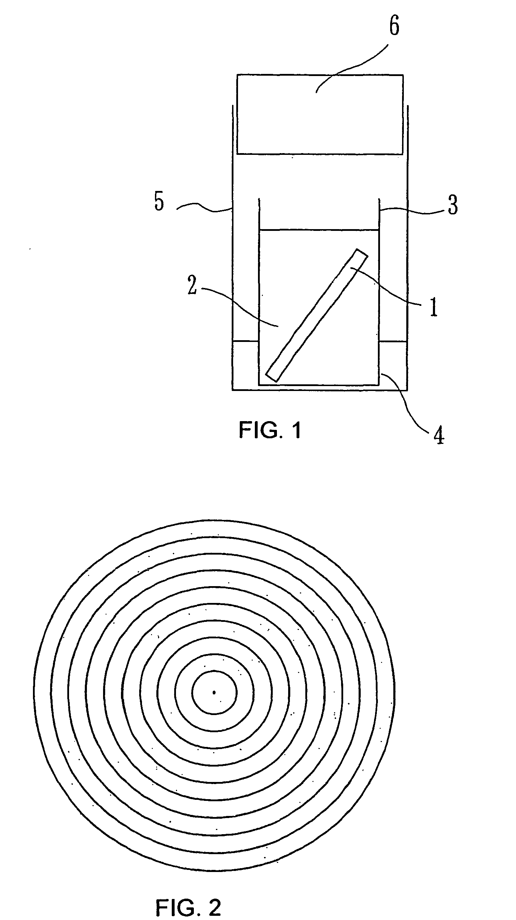

In the present invention, a fine spherical particle having uniform molecular orientation means that the fine particle has a nearly spherical shape and is evenly oriented in a radial pattern from the center, i.e., it has concentric molecular orientation having point disclination. Fig. 2 shows one example of the schematic drawing of a fine spherical particle having such molecular orientation. When observed on a polarizing microscope, such a fine particle shows a crisscross on the spherical body under cross-Nicol and the even orientation can be confirmed from the fact that the direction of the crisscross does not change even when the object is rotated.

The fine spherical particle having uniform molecular orientation can be obtained as a fine particle having a particle diameter of about 0.01 to 1000 µm but a desired particle diameter can be obtained by adjusting production conditions. When the fine spherical particle of the present invention is used in fine chemical fields and electronic and information fields, such as a functional material and a medical material, it is preferred that the particle diameter is about from 0.01 to 100 µm, particularly about from 0.05 to 50 µm.

The fine spherical particle of the present invention can be produced by immersing a substrate having hydrophilicity in an aqueous solution of a salt of the compound represented by the following formula (1) and precipitating the fine particle under an acidic atmosphere: 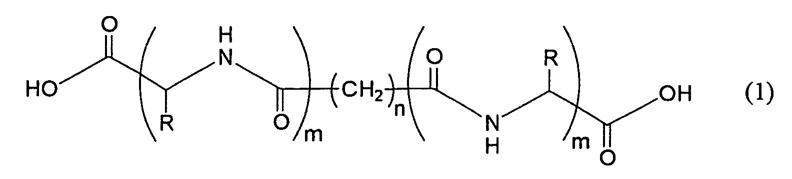 wherein R represents a hydrogen atom or a linear or branched alkyl group having 1 to 5 carbon atoms, such as methyl, ethyl, n-propyl, i-propyl, and butyl; n is an integer of 8 to 20; and m is an integer of 1 to 3.

Preferred are compounds wherein, in formula (1), R is a hydrogen atom, a methyl group, or an isopropyl group; m is 2; and n is 8, 10, or 12. Of these, more preferred is a compound wherein R is an isopropyl group; n is 10; and m is 2.

The metal salts of these compounds are not particularly limited, so long as they are water-soluble metal salts, and various metal salts such as alkali metal salts and alkaline earth metal salts can be used, but it is preferred to use a sodium or potassium salt.

The concentration of the salt of the compound represented by formula (1) in the aqueous solution is preferably about from 0.1 to 100 mM/L, particularly about from 0.5 to 20 mM/L.

The substrate to be immersed in the aqueous solutions of these salts is not particularly limited, so long as it has hydrophilicity, and examples include glass, metal, silica, mica, ceramic, earthenware, porcelain, plastic, and a composite material thereof. When the substrate has no hydrophilicity, it is suitable to impart hydrophilicity by subjecting the surface to a hydrophilicity treatment. The hydrophilicity treatment can be suitably selected according to the properties of the substrate, but when the substrate is glass, for example, the treatment can be carried out by washing the surface of the glass substrate and subsequently immersing it in a solution wherein an alkali such as potassium hydroxide is dissolved in an alcohol such as ethanol.

The shape of the substrate is not particularly limited, and the substrate having any shape such as plate-like one or tubular one can be used. Moreover, the substrate having not only a smooth surface but also a surface which is not smooth can be also used.

The fine spherical particle of the present invention can be precipitated and grown by maintaining the aqueous solution of the compound (1), in which the substrate having hydrophilicity is immersed, in an acidic atmosphere, preferably a weakly acidic atmosphere of about pH 4 to 6.

The maintenance of the aqueous solution of the compound (1), in which the substrate having hydrophilicity is immersed, in an acidic atmosphere is carried out by placing a vessel 3 containing an aqueous solution 2 of the compound (1), in which the substrate 1 is immersed, in a vessel 5 containing an aqueous acidic solution 4 of acetic acid or the like and sealing the whole system by capping the vessel 5 with a lid 6, as shown in Fig. 1.

The fine particles precipitated on the surface of the substrate can be exfoliated and recovered from the substrate by ultrasonic or mechanical means.

### Spherical microcapsule:

Moreover, the present invention includes a method for producing a microcapsule encapsulating a core substance inside the microcapsule utilizing membrane formation by self-assembly of the above bicephalic amphiphilic compound represented by formula (1).

The process for producing the spherical microcapsule according to the present invention comprises forming a spherical membrane having uniform molecular orientation to obtain a spherical microcapsule having a nearly even particle diameter by chemical and physicochemical methods without using conventional techniques such as polymerization, curing, and lamination.

In the present invention, by preparing an aqueous solution in which a metal salt of the above compound and a hydrophilic core substance are dissolved, immersing a hydrophilicity-treated substrate in the aqueous solution, and subsequently allowing the aqueous solution to stand under an acidic atmosphere for a certain period of time, the carboxylate metal salt of the above compound gradually changes into the corresponding carboxylic acid and the compound becomes insoluble, whereby spherical microcapsules encapsulating fine particles of the hydrophilic core substance precipitate on the surface of the substrate. The concentration of the metal salt in the aqueous solution is in the range of 0.1 to 100 mM/L, preferably 0.5 to 20 mM/L

The particle diameter of each of the spherical microcapsules thus formed is preferably from 0.01 to 100 µm, more preferably from 0.05 to 50 µm.

In the present invention, the hydrophilic core substance to be encapsulated in the microcapsule is not particularly limited, so long as it is a water-soluble chemical substance and examples thereof include dyes, pharmaceutical compounds, fragrant materials, pesticide compounds, foods, and the like. Moreover, as the substrate, a substrate board or the like selected from glass, metal, silica, mica, ceramic, earthenware, porcelain, plastic, and a composite material thereof and subjected to a hydrophilicity treatment to make the surface hydrophilic is immersed in the above aqueous solution beforehand and then used. Furthermore, the above aqueous solution is allowed to stand under an acidic atmosphere. At that time, the solution is preferably allowed to stand for several days under a weakly acidic atmosphere of pH 5 to 6 using an aqueous solution of a weak acid such as acetic acid as the acidic atmosphere.

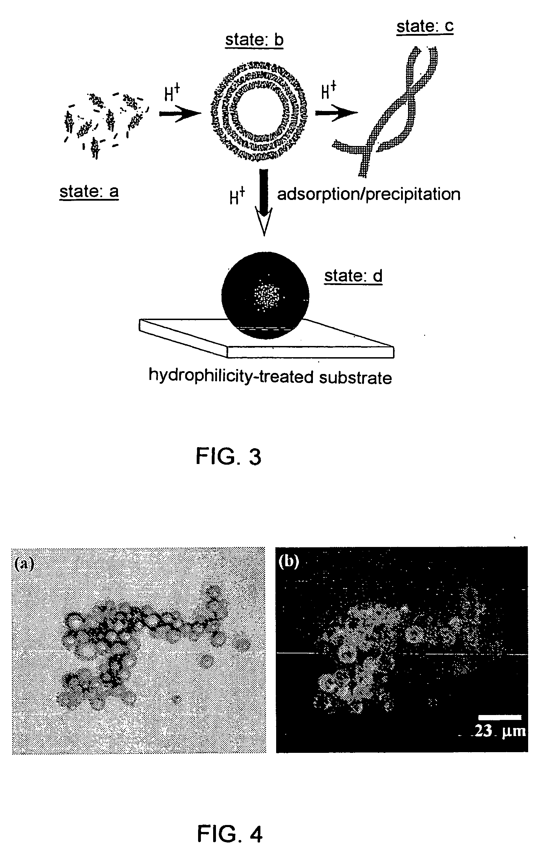

The following will describe the production process of the microcapsule in the present invention, mainly the mechanism of self-assembly of the compound represented by formula (1) (hereinafter referred to as "compound X") with reference to Fig. 3.

The metal salt of the compound X is water-soluble and dissolves in water to form rod-like micelles already in the metal salt state (state: a). Then, protonation of the metal salt of the compound X gradually proceeds under a weakly acidic atmosphere. Thereby, solubility of the compound X in water decreases and the compound becomes insoluble. With the progress of insolubilization of the compound X (conversion into carboxylic acid), the compound X aggregates to form a fibrous structure (state: c) via a spherical multiplayer vehicle intermediate (state: b). The change of the structural form from the state (a) to the state (c) can be confirmed by the analysis in accordance with a light scattering method using a laser light.

The state (b) is an intermediate to the state (c) and is a state dispersed in water. The protonation is not completed and only some carboxylic acids are protonated in most cases. Therefore, it is suggested that the terminals in the metal salt state cover the uppermost surface of the vehicle (state: b). Moreover, the state (b) can be first obtained by immersing the hydrophilic substrate in the system.

The intermediate vehicle is trapped on the surface of the substrate by the interaction with the substrate and protonation proceeds as it stands on the surface, whereby the vehicle precipitates as a hollow spherical body (d) without the conversion into the state (c). The thus precipitating spherical body can be easily confirmed using an optical microscope, an electron microscope, or the like.

### Industrial Applicability

According to the present invention, a fine spherical particle having uniform molecular orientation or a stable spherical microcapsule encapsulating a water-soluble core substance can be inexpensively and easily obtained by membrane formation through self-assembly of a specific bicephalic compound without requiring complicated production steps, such as conventional polymerization, and special apparatus.

The fine spherical particle and spherical microcapsule obtained in the present invention can be used in medical and pharmaceutical fields as biocompatible materials and in fine chemical fields such as pharmaceuticals, cosmetics, and dyes as supports for supporting active ingredients or the like. Furthermore, they are usable as various optical materials, functional materials, and the like utilizing the polarizing properties.

### Examples

The present invention is described below in more detail with reference to Examples, but the present invention is not limited to these Examples.

### Example 1

### Process for producing fine spherical particle:

An aqueous solution was obtained wherein [bis(N-α-amino-L-valine-L-valine) 1,10-decane]dicarboxylic acid (compound wherein m is 2; n is 10; and R is an isopropyl group in formula (1)) represented by the following structural formula A was dissolved by using 2 equivalents of an aqueous sodium hydroxide solution to give a concentration of 1 mM/L

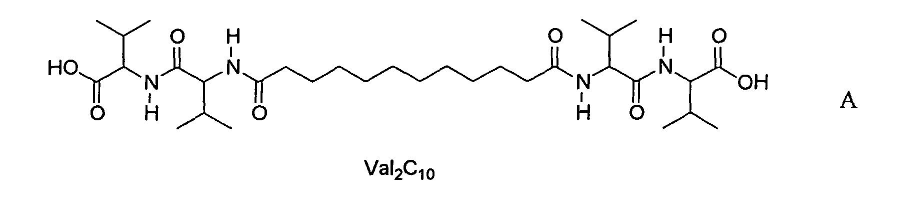

A glass substrate board as a substrate was immersed in a detergent for 3 hours, followed by ultrasonic cleaning (20 minutes × 4 times) using ultrapure water (Milli-Q). Then, ultrasonic cleaning was carried out by using an aqueous solution of 2% Extran MA-02 (blue label, neutral) for 20 minutes, followed by ultrasonic cleaning (20 minutes × 4 times) using ultrapure water (Milli-Q). The substrate was immersed for 4 hours in a solution obtained by dissolving 60 g of potassium hydroxide in 700 ml of ethanol, followed by ultrasonic cleaning (20 minutes × 4 times) using ultrapure water to prepare a hydrophilicity-treated substrate.

Then, as shown in Fig. 1, a vessel wherein the hydrophilicity-treated substrate was immersed in the above aqueous solution of the compound A was placed in a vessel containing a weakly acidic solution which was an aqueous solution of 5% acetic acid (v/v) and the vessel was capped, whereby the whole system was allowed to stand under an acidic atmosphere.

When the vessel was allowed to stand at room temperature or in a cold place for 2 to 3 days, it was visually confirmed that fine spherical particles precipitated on the surface of the substrate and the substrate became clouded. The fine spherical particles were recovered by treating the substrate with an ultrasonic wave.

The resulting fine particles were spherical bodies having uniform particle diameter of 5 to 15 µm. When the fine particles were observed on a polarizing microscope, a crisscross was confirmed on each of the spherical bodies under cross-Nicol. Also, it was confirmed that the direction of the crisscross was not changed even when the object was rotated and it was evenly oriented, i.e., it has concentric molecular orientation having point disclination.

### Example 2

### Process for producing spherical microcapsule:

An aqueous solution was obtained wherein [bis(N-α-amino-L-valine-L-valine) 1,10-decane]dicarboxylic acid represented by the above structural formula A was dissolved by using 2 equivalents of an aqueous sodium hydroxide solution to give a concentration of 1 mM/L.

A glass substrate board subjected to the following hydrophilicity treatment was used as a substrate. First, the board was immersed in a detergent overnight and then ultrasonic cleaning was carried out for 1 hour. Then, ultrasonic cleaning (20 minutes x 4 times) was carried out using ultrapure water (Milli-Q). Furthermore, ultrasonic cleaning was carried out by using an aqueous solution of 2% Extran MA-02 (blue label, neutral) for 20 minutes, followed by ultrasonic cleaning (20 minutes x 4 times) using ultrapure water (Milli-Q). The substrate was immersed for 4 hours in a solution obtained by dissolving 60 g of potassium hydroxide in 700 ml of ethanol, followed by ultrasonic cleaning (20 minutes × 4 times) using ultrapure water to prepare a hydrophilicity-treated substrate.

Then, 8-hydroxypyren-1,3,6-trisulfonic acid sodium salt (pyranine) represented by the following structural formula as an substance B to be encapsulated was dissolved to give a concentration of 10 mM in the aqueous solution in which the compound A was dissolved.

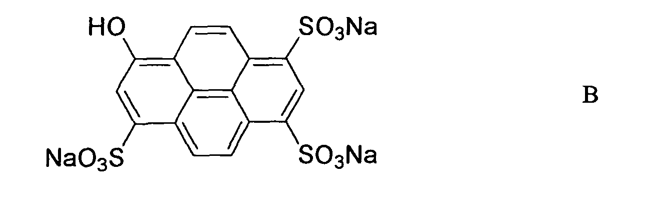

A vessel wherein the glass hydrophilicity-treated substrate was immersed in the above aqueous solution containing the compounds A and B was placed in a vessel containing a weakly aqueous acidic solution which was an aqueous solution of 5% acetic acid (v/v) and the vessel was capped, whereby the whole system was allowed to stand under an acidic atmosphere, as shown in Fig. 1. Thereafter, when the vessel was allowed to stand at room temperature or in a cold place for 2 to 3 days, spherical microcapsules incorporating the inclusion substance B precipitated on the surface of the substrate.

The resulting fine particles were spherical microcapsules having uniform particle diameter of 5 to 15 µm. When the microcapsules were observed on a fluorescent microscope, it was confirmed that the microcapsule was a microcapsule emitting fluorescence owing to pyranine as the inclusion compound (cf. Fig. 4).

## Claims

1. A fine spherical particle having uniform molecular orientation, which comprises a compound represented by the following formula (1): 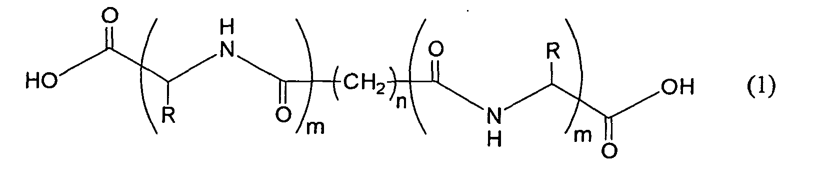 wherein R represents a hydrogen atom or an alkyl group having 1 to 5 carbon atoms; n is an integer of 8 to 20; and m is an integer of 1 to 3.

2. The fine spherical particle according to claim 1, wherein the fine particle is evenly oriented in a radial pattern from the center.

3. The fine spherical particle according to claim 1 or 2, wherein the particle diameter of the fine particle is from 0.01 to 100 µm.

4. A process for producing the fine spherical particle according to any one of claims 1 to 3, which comprises immersing a substrate having hydrophilicity in an aqueous solution of a salt of the compound represented by formula (1) to precipitate the fine particle under an acidic atmosphere.

5. The process for producing the fine spherical particle according to claim 4, wherein the salt of the compound represented by formula (1) is an alkali metal salt.

6. The process for producing the fine spherical particle according to claim 4 or 5, wherein the substrate comprises glass, metal, silica, mica, ceramic, earthenware, porcelain, plastic, or a composite material thereof.

7. The process for producing the fine spherical particle according to any one of claims 4 to 6, wherein the fine particle is precipitated under an acidic atmosphere of pH 5 to 6.

8. A spherical microcapsule in which a fine particle of a hydrophilic core substance are encapsulated inside the spherical body of the compound represented by formula (1) having uniform molecular orientation.

9. The spherical microcapsule according to claim 8, wherein the spherical microcapsule has a particle diameter of from 0.01 to 100 µm.

10. A process for producing the spherical microcapsule encapsulating a fine particle of a hydrophilic core substance according to claim 8 or 9, which comprises immersing a hydrophilicity-treated substrate in an aqueous solution in which a metal salt of the compound represented by formula (1) and the hydrophilic core substance are dissolved; and allowing the aqueous solution to stand under an acidic atmosphere for precipitation.

11. The process for producing the spherical microcapsule according to claim 10, wherein the metal salt of the compound represented by formula (1) is an alkali metal salt.

12. The process for producing the spherical microcapsule according to claim 10 or 11, wherein the acidic atmosphere is a weakly acidic atmosphere of pH 5 to 6.

13. The process for producing the spherical microcapsule according to any one of claims 10 to 12, wherein the substrate is selected from glass, metal, silica, mica, a ceramic, earthenware, porcelain, plastic, and a composite material thereof.
